# EUROPÄISCHE PATENTSCHRIFT

(11) **EP 3 980 093 B1**
(45) Veröffentlichungstag und Bekanntmachung des Hinweises auf die Patenterteilung: **14.12.2022**
(21) Anmeldenummer: 20732150.6
(22) Anmeldetag: 04.06.2020
(51) Int. Cl.: A61M 1/36

(54) **DRUCKMESSERANORDNUNG FÜR EINE EXTRAKORPORALE BLUTBEHANDLUNGSMASCHINE**
PRESSURE-MEASURING ASSEMBLY FOR AN EXTRACORPOREAL BLOOD TREATMENT MACHINE
ENSEMBLE DE MESURE DE PRESSION DESTINÉ À UNE MACHINE DE TRAITEMENT DE SANG EXTRACORPORELLE

(30) Priorität: 06.06.2019 DE 102019115271
(43) Veröffentlichungstag der Anmeldung: 13.04.2022
(73) Patentinhaber: B. Braun Avitum AG, 34212 Melsungen (DE)
(72) Erfinder: RITTER, Kai-Uwe, 34212 Melsungen (DE)
(74) Vertreter: Winter, Brandl - Partnerschaft mbB
(86) Internationale Anmeldenummer: PCT/EP2020/065477
(87) Internationale Veröffentlichungsnummer: WO 2020/245267

(56) Entgegenhaltungen:
- EP-A2- 1 843 140
- US-B2- 8 210 049

## Beschreibung

Die vorliegende Erfindung betrifft eine Druckmessanordnung zur Messung des Leitungsinnendrucks eines vorzugsweise extrakorporalen Leitungssystems einer extrakorporalen Blutbehandlungsmaschine, insbesondere Dialysemaschine, mit einem Drucksensor, der über eine starre, biegesteife Fluidleitung, vorzugsweise Gasleitung, mit einem an das Leitungssystem angeschlossenen oder anschließbaren Druckabnehmer verbindbar ist und ein Fluiddrucksignal aus der starren, biegesteifen Fluidleitung in ein elektrisches Signal wandelt, einer elektrische Leitung, vorzugsweise Kabel, die dazu dient, den Drucksensor mit einer Elektronik zu verbinden, um das elektrische Signal zu verarbeiten, sowie einer Halterung zum direkten oder indirekten Halten des Drucksensors. Ferner betrifft die vorliegende Erfindung eine extrakorporale Blutbehandlungsmaschine mit einer entsprechenden Druckmessanordnung.

### Stand der Technik

Extrakorporale Blutbehandlungsmaschinen, insbesondere Dialysemaschinen, weisen ein Leitungssystem, insbesondere mit einem Dialyseflüssigkeitskreislauf sowie einem extrakorporalen Blutkreislauf, auf. Diese Kreisläufe haben eine Anzahl von Dialyseflüssigkeit- und Blut(schlauch)leitungen, welche diverse Funktionseinheiten der extrakorporalen Blutbehandlungsmaschine, wie z.B. einen Dialysator, Blutpumpen etc., durchlaufen und/oder verbinden. Um eine reibungslose Funktionalität der extrakoralen Blutbehandlungsmaschine zu gewährleisten, muss dieses Leitungssystem, insbesondere die Blutschlauchleitungen, an mehreren Stellen drucküberwacht werden. Beispielsweise sind bei einer gängigen Dialysemaschine ein PA-Anschluss zur Überwachung des arteriellen Unterdrucks, ein PBE-Anschluss zur Überwachung des Eingangsdrucks vor dem Dialysator und ein PV-Anschluss zur Überwachung des venösen Drucks bereitgestellt.

Zum Zweck der Drucküberwachung sind als Druckabnehmer z.B. Abzweige oder sogenannte PODs (Oszillierenden Druckmembrane) an geeigneten Stellen in den Leitungen oder Blutschlauchleitungen eingebaut, welche nachfolgend auch als Druckabnehmerkapsel bezeichnet werden. Entsprechende PODs oder Druckabnehmerkapseln sind aus dem Stand der Technik bekannt. Beispielsweise offenbaren US 8 092 414 B2 und US 8 491 518 B2 Druckabnehmerkapseln mit einer volumenstarren Kapsel, welche durch eine Membran in zwei Kammern geteilt ist, genauer in eine erste, an ein Leitungssystem angeschlossene Kammer sowie eine zweite (luftseitige) Kammer, welche über einen dünnen Schlauch mit einem Drucksensor oder einem zugehörigen Anschluss verbunden ist.

In anderen Worten ausgedrückt, sind die Abzweige oder PODs mittels dünner Schläuche an Druckabnehmeranschlüssen (Druckaufnehmeranschlüssen) mit der Maschine (extrakorporalen Blutbehandlungsmaschine), genauer, mit einem Gehäuse der Maschine, verbunden oder daran angeschlossen. Die Anschlüsse sind dabei typischerweise mit Luer-Lock-Konnektoren versehen. Da häufig Sensoren verwendet werden, welche nicht dazu geeignet sind, um direkt durch ein Gehäuse nach außen geführt zu werden, ist ggf. ein zusätzlicher, gehäuseinterner Schlauch notwendig, um den Druckabnehmeranschluss des Gehäuses mit dem in dem innerhalb des Gehäuses angeordneten Drucksensor zu verbinden. Alternativ sind Drucksensoren bekannt, welche über einen dünnen Schlauch direkt mit einem Luer-Lock/Slip-Konnektor versehen sind.

Dadurch, dass die Druckabnehmerkapseln in den Schlauchleitungen des Leitungssystems hängen und somit an nicht genau definierten, instabilen Positionen schweben, können Messungenauigkeiten entstehen. Aus US 9 393 397 B2 ist bekannt, dieses Problem zu vermeiden, indem die Druckabnehmerkapsel unmittelbar an einem Dialysator angebracht wird. Probleme, welche durch den dünnen Schlauch selbst entstehen, können dadurch jedoch nicht vermieden werden. Das heißt, dies hat immer noch den Nachteil der langen Schlauchverbindung zwischen POD (Druckabnehmer) und Druckabnehmeranschluss (Druckaufnehmeranschluss). Beispielsweise ist der dünne Schlauch anfällig für Beschädigungen wie ein Abknicken durch einen unachtsamen Anwender. Ferner sind eine Vielzahl von Übergängen oder Anschlussstellen notwendig, insbesondere, falls ein zusätzlicher, gehäuseinterner Schlauch vorgesehen ist, was Undichtigkeiten in der Leitung begünstigt und zudem zu einer aufwändigen, teuren Montage führt. Des Weiteren ist es für einen Anwender einfach, den Schlauch, welcher über eine standardisierte Schnittstelle wie einen Luer-Lock-Konnektor an einem Druckabnehmeranschluss des Gehäuses anzubringen ist, versehentlich an einem falschen Anschluss zu konnektieren. Ein solches falsches Konnektieren kann zu Unterbrechungen oder Verzögerungen einer Therapie, insbesondere einer Dialysetherapie, führen und schlimmstenfalls Patienten oder Personal gefährden.

Im Betrieb wird ein in dem Leitungssystem und somit in der ersten Kammer anliegender Leitungsinnendruck über die Membran auf die zweite Kammer übertragen, in welcher somit ein von dem Leitungsinnendruck abhängiger Fluiddruck, insbesondere Gasdruck, entsteht. Der mit der zweiten Kammer über den dünnen Schlauch verbundene Drucksensor erhält somit ein Fluiddrucksignal, welches er in ein elektrisches Signal wandelt. Durch den dünnen, flexiblen Schlauch, welcher eine große Fluid- oder Luftstrecke und ein Totvolumen zwischen der Druckabnehmerkapsel und dem Drucksensor bildet, entsteht eine Tiefpasswirkung, sodass nachteiliger Weise die Genauigkeit der Messung und eine Reaktionszeit des Drucksensors eingeschränkt ist.

Aus US 8 210 049 B2 und EP 1 843 140 A2 sind Druckabnehmerkapseln oder PODs bekannt, bei welchen zur Verbesserung der Messgenauigkeit die Druckabnehmerkapsel unmittelbar oder über eine volumenstarre, kurze Verbindung mit dem Drucksensor verbunden ist. Dabei kann der Drucksensor an einem Rahmen einer permanenten Halterung angebracht sein. Falls eine solche Halterung beispielsweise unmittelbar in einem Gehäuse einer extrakorporalen Blutbehandlungsmaschine bereitgestellt ist, kann eine Montage des Leitungssystems umständlich und aufwändig sein. Insbesondere kann eine Verwendung unterschiedlicher Leitungssysteme oder Blutschlauchleitungen erschwert oder sogar unmöglich sein. Das heißt, die Halterung ist voraussichtlich nicht darauf ausgelegt oder ausreichend flexible, dass eine Anzahl unterschiedlicher Leitungssysteme daran anschließbar sind. Falls die permanente Halterung außerhalb des Gehäuses, z.B. an einem Dialysator oder an einer Blutpumpe bereitgestellt ist, muss eine elektrische Leitung zwischen der Halterung und einer signalverarbeitenden Elektronik bereitgestellt werden, welche durch Umgebungseinflüsse wie unvorsichtige Anwender, aggressive Desinfektionsmittel etc. leicht beschädigt werden können und ist eine Position des Drucksensors nur eingeschränkt wählbar.

### Zusammenfassung der Erfindung

Die der vorliegenden Erfindung zugrunde liegende Aufgabe besteht darin, Nachteile des Stands der Technik zu verbessern oder zu beseitigen. Insbesondere soll eine zur Verwendung unterschiedlich ausgebildeter Leitungssysteme ausgelegte/geeignete Druckmessanordnung für eine extrakorporale Blutbehandlungsmaschine bereitgestellt werden, die ein falsches Anschließen von Druckabnehmern des Leitungssystems unmöglich macht und ein funktionssicheres und präzises Messen eines Leitungsinnendrucks ermöglicht.

Die der Erfindung zugrunde liegende Aufgabe wird durch eine Druckmessanordnung mit den Merkmalen des Anspruchs 1 gelöst. Vorteilhafte Ausgestaltungen sind Gegenstand der Unteransprüche und werden später genauer beschrieben.

Der Kerngedanke der Erfindung besteht darin, eine Druckmessanordnung zur Messung eines Leitungsinnendrucks einer Leitung/eines Leitungssystems einer extrakorporalen Blutbehandlungsmaschine bereitzustellen, welche eine maschinenexterne Halterung, die bevorzugt 15 bis 30 cm lang ist, zum Halten eines Drucksensors und eines Druckabnehmers der Leitung/des Leitungssystems, welche aneinander über eine starre, insbesondere volumenkonstante Fluidleitung anschließbar sind, bereitstellt. Die Halterung ist dazu ausgebildet, den Drucksensor direkt oder indirekt (z.B. über den Druckabnehmer) derart zu halten, dass eine elektrische Verbindung durch die Halterung selbst vor Umgebungseinflüssen geschützt ist und/oder dass eine Position des Drucksensors derart eingestellt oder einstellbar ist, dass unterschiedliche Leitungen/Leitungssystemen einfach mit der bereitgestellten Druckmessanordnung verbindbar bzw. verwendbar sind.

Genauer ausgedrückt, wird die der Erfindung zugrunde liegende Aufgabe durch eine Druckmessanordnung zur Messung des Leitungsinnendrucks eines vorzugsweise extrakorporalen Leitungssystems (an einer bestimmen Stelle/Leitungsabschnitt desselben) einer extrakorporalen Blutbehandlungsmaschine, insbesondere Dialysemaschine, gelöst, mit einem Drucksensor, der über eine starre, (plastisch und im Wesentlichen auch elastisch) biegesteife (insbesondere volumenkonstante) Fluidleitung, vorzugsweise Gasleitung, mit einem an das Leitungssystem angeschlossenen oder anschließbaren Druckabnehmer verbindbar ist und ein Fluiddrucksignal aus der starren, biegesteifen Fluidleitung in ein elektrisches Signal wandelt, mit einer elektrische Leitung, vorzugsweise einem Kabel, die dazu dient, den Drucksensor mit einer Elektronik zu verbinden, um das elektrische Signal zu verarbeiten, sowie mit einer Halterung zum direkten oder indirekten Halten des Drucksensors.

Die Halterung der vorstehenden Druckmessanordnung ist als ein im Wesentlicher plastisch (insbesondere durch einen Anwender) abkrümmbarer Stab ausgebildet, der an einem Axialendabschnitt eine Befestigungsvorrichtung zur Montage an einem ortsfesten Untergrund und an dem anderen Axialendabschnitt eine Anlenkstelle für den Drucksensor oder den Druckabnehmer aufweist. In anderen Worten ausgedrückt, hat die Halterung eine Längserstreckung, welche es ermöglicht, den Drucksensor an einer optimal ausgewählten oder einstellbaren Position außerhalb des Gehäuses der extrakorporalen Blutbehandlungsmaschine zu halten. Vorzugsweise ist der Stab biegestarr ausgebildet, was in diesem Zusammenhang bedeutet, dass die Halterung/der Stab sich gegenüber dem Eigengewicht des Drucksensors und dem daran angeschlossenen Druckabnehmer und gegebenenfalls der Leitungen sowie vorzugsweise unter einem anwendungsbedingten Vibrieren nicht oder nur minimal elastisch und nicht plastisch biegt, um eine festgelegte Positionierung des Drucksensors im Raum bereitzustellen. Andererseits ist der Stab, wie vorstehend beschrieben, unter einer höheren, insbesondere für einen Anwender möglichen Kraftanwendung plastisch verformbar. Alternativ oder zusätzlich zu der Ausbildung als (biegestarrer) plastisch abkrümmbarer Stab, wie vorstehend beschrieben, bildet die Halterung zumindest abschnittsweise einen Innenkanal, in welchem die elektrische Leitung in vor der Umgebung geschützter Weise geführt ist. D. h., eine Wandung des Innenkanals bzw. eine Struktur oder ein Material der Halterung ist derart gewählt, dass diese stabiler, insbesondere härter und/oder biegesteifer als die elektrische Leitung (d.h., es wird eine größere Kraft benötigt, um die Halterung zu verbiegen als die elektrische Leitung) und vorzugweise beständig gegenüber Umgebungseinflüssen wie aggressiven Desinfektionsmitteln ist.

In der vorstehend beschriebenen Druckmessanordnung wird kein zusätzlicher dünner Schlauch zur Verbindung des Druckabnehmers benötigt, was Kosten spart, da ein solcher Schlauch in der Regel als Einwegteil bereitgestellt wird. Ferner ist somit eine Anzahl von Schnittstellen, die Leckage unterliegen können, gering und ist ein fehlerhaftes Anschließen des dünnen Schlauchs ausgeschlossen. Zudem ist von Vorteil, dass das Totvolumen der sonst vorhandenen Schläuche verringert werden kann und eine Tiefpasswirkung, welche durch die Luftstrecke in einem solchen Schlauch entsteht, verhindert werden kann. Insbesondere ist es vorteilhaft, wenn die starre, biegesteife Fluidleitung zwischen dem Druckabnehmer und dem Drucksensor möglichst kurz gehalten ist. Demgemäß ist es möglich, eine genauere Druckmessung und kürzere Reaktionszeiten des Drucksensors auf Druckänderungen des Leitungssystems, insbesondere in einer Blutschlauchleitung der extrakorporalen Blutbehandlungsmaschine, zu gewährleisten. Ferner ist von Vorteil, dass der Drucksensor gehäuseextern an einer geeigneten, vorzugsweise einstellbaren Position gehalten werden kann und/oder dass eine Beschädigung der elektrischen Leitung aufgrund des schützenden Innenkanals vermeidbar ist.

Zum Anschließen des Drucksensors über die starre, biegesteife Fluidleitung an den Druckabnehmer ist ein Koppelabschnitt, vorzugsweise ein Luer-Lock/Slip-Konnektor oder eine Klick-Verbindung/einrastbares Verbindung, bereitgestellt. Dabei handelt es sich um besonders einfache, kostengünstige und einfach zu bedienende Anschlüsse. Ferner wird bevorzugt die starre, biegesteife Fluidleitung durch den Koppelabschnitt gebildet, um ein Innenvolumen der starren, biegesteifen Fluidleitung (ihrer Länge) möglichst gering (kurz) zu halten. Dies erhöht weiter die Messgenauigkeit.

Um die elektrische Leitung besonders effektiv zu schützen, ist es von Vorteil, wenn der Innenkanal einen zur Umgebung hin vollständig geschlossenen Aufnahmeraum für die elektrische Leitung bildet.

Die Halterung kann den Drucksensor direkt halten, was es ermöglicht, bei einer Vorbereitung einer extrakorporalen Blutbehandlung den Druckabnehmer des Leitungssystems unter Nutzung einer einzigen Schnittstelle mit dem Drucksensor zu verbinden. Dies gewährleistet demgemäß eine besonders einfache und schnelle Montage des Leitungssystems. Alternativ kann die Halterung den Drucksensor indirekt halten, beispielsweise über den Druckabnehmer. D.h., zur Montage des Leitungssystems wird zunächst der Druckabnehmer an der Halterung angebracht und anschließend der Drucksensor über die starre, biegesteife Fluidleitung an den Druckabnehmer angeschlossen. Es ist denkbar, die Halterung hierfür derart auszubilden, dass eine elektrische Verbindung beim Anschließen des Drucksensors entsteht, beispielsweise über einen Berührungskontakt, Induktion oder eine Steckverbindung.

Vorzugsweise ist die Halterung derart plastisch verformbar oder abkrümmbar, weiter vorzugsweise in Form eines Schwanenhalses, ausgebildet, sodass eine Position der Anlenkstelle vorzugsweise manuell einstellbar ist. Dies ermöglicht es, den an der Anlenkstelle gehaltenen Drucksensor während der Vorbereitung der Dialysebehandlung aus dem Weg zu schieben bzw. sie entsprechend zu verformen und bei der Montage des Leitungssystems an eine zum Anschließen des Druckabnehmers geeignete Position einzustellen. D. h., die Halterung ist im Rahmen der Montage des Leitungssystems einfach an unterschiedlich ausgebildete Leitungssysteme anpassbar. Man kann auch sagen, die Halterung nach dieser Ausführungsform ist ein halbsteifes, biegsames Verbindungselement, beispielsweise aus einem gewendelten Material (z.B. Metallschlauch), welches in beinahe jede Richtung gebogen werden und in jeder beliebigen Stellung verharren kann. Insbesondere ist es von Vorteil, wenn der Innenkanal durch den Schwanenhals ausgebildet ist. Auf diese Weise ist es möglich, den Drucksensor über den Schwanenhals passend zu verstellen/einzustellen, ohne dadurch die elektrische Leitung zu beschädigen und diese zudem gegen Umgebungseinflüsse schützen.

Alternativ kann die Halterung ein starrer, in einer vorbestimmten, linearen oder gekrümmten Form verlaufender, vorzugsweise hohler Stab sein. Nach dieser Ausführungsform kann die Halterung derart geformt sein, dass die Anlenkstelle an einer zum Anschließen des Leitungssystems geeigneten Position angeordnet ist und eine Gefahr einer ungenauen Messung, dadurch, dass sich die Position des Drucksensors während einer Dialysebehandlung (nach einer Kalibrierung des Drucksensors) ändert, im Wesentlichen vollständig verhindert werden kann.

Es ist von Vorteil, wenn der Druckabnehmer nach Art einer Druckabnehmerkapsel ausgebildet ist, die eine erste, mit der Leitung/dem Leitungssystems verbundene oder verbindbare Kammer und eine zweite, vorzugsweise gasgefüllte Kammer aufweist, welche von der ersten Kammer durch eine Membran getrennt ist und an welche der Drucksensor gekoppelt oder koppelbar ist. In anderen Worten ausgedrückt, ist der Druckabnehmer als eine Druckabnehmerkapsel ausgebildet, welche es ermöglicht, einen Leitungsinnendruck zuverlässig zu messen, ohne eine Sterilität zum Beispiel einer zu vermessenden Blutleitung zu gefährden. Bevorzugter Weise ist in diesem Fall ein an dem Drucksensor oder ein an der starren, biegesteifen Fluidleitung integriertes oder montiertes Ventil bereitgestellt, um die Druckabnehmerkapsel, insbesondere die zweite Kammer, zu be- oder entlüften. Das heißt, das Ventil ist mit der zweiten Kammer der Druckabnehmerkapsel bzw. mit der starren, biegesteifen Fluidleitung fluidisch verbunden. Dies ermöglicht es, die Membran der Druckabnehmerkapsel in eine bestimmte Stellung zu bringen, in dem das Ventil geöffnet wird und die Membran durch eine Druckänderung z.B. auf der Blutseite ausgerichtet wird. Anschließend wird das Ventil wieder hermetisch geschlossen. D. h. das Ventil ist dem Drucksensor zugeordnet und ist somit als Mehrwegteil bereitgestellt. Die in der Regel als Einwegteil bereitgestellte Druckabnehmerkapsel benötigt demgemäß kein Ventil und kann kostengünstiger gefertigt werden.

Vorzugsweise hält die Halterung den Drucksensor, hält der Drucksensor die starre biegesteife Fluidleitung (oder ist daran, insbesondere über den Koppelabschnitt, befestigt), und ist die Fluidleitung dazu eingerichtet, an dem Druckabnehmer befestigt zu werden. In anderen Worten ausgedrückt, sind die Halterung, der Drucksensor und die starre, biegesteife Fluidleitung sowie ggf. der Druckabnehmer in dieser Reihenfolge aneinander angeordnet bzw. miteinander verbunden. Insbesondere sind diese Elemente Teil folgender Signalübertragungseinrichtung. Wenn ein Druckabnehmer an der starren, biegesteifen Fluidleitung angeschlossen ist, wird ein Drucksignal (insbesondere ein Gas-/Luftdruck) über die starre, biegesteife Fluidleitung zu dem Drucksensor geleitet. Bevorzugt ist die Fluidleitung (unmittelbar) an dem Drucksensor angeordnet (verbindet den Druckabnehmer und den Drucksensor) und ist durchgängig starr und biegesteif ausgebildet. Das Drucksignal wird durch den Drucksensor in ein elektrisches Signal gewandelt. Von dem Drucksensor wird das elektrische Signal auf eine elektrische Leitung übertragen, die durch den Innenkanal der Halterung verläuft und die dazu ausgebildet ist, das elektrische Signal von dem Drucksensor zu einer Steuerungseinheit zu übertragen.

Die Steuerungseinheit ist insbesondere (geschützt) in einem Innenraum eines Gehäuses aufgenommen. Die Halterung, der Drucksensor und die starre, biegesteife Fluidleitung sind insbesondere an einem nach außen gewandten Teil des Gehäuses angeordnet. Bevorzugt ist die Halterung dazu eingerichtet (insbesondere außenseitig / zu einem Gehäuseäußeren gewandt) an einem Gehäuseabschnitt der extrakorporalen Blutbehandlungsmaschine befestigt zu werden. In anderen Worten ausgedrückt, ist bevorzugt ein dem Drucksensor gegenüberliegendes Ende der Halterung an einer (dem Gehäuseinnenraum abgewandten/nach außen ausgerichteten) Seite des Gehäuseabschnitts, insbesondere einer Gehäuseplatte, befestigt und ist weiter bevorzugt die Steuerungseinheit an einer entgegengesetzten (dem Gehäuseinnenraum zugewandten) Seite des Gehäuseabschnitts angeordnet. Die elektrische Leitung verläuft von dem Drucksensor durch die Halterung zu dem Gehäuseabschnitt, vorzugsweise durch den Gehäuseabschnitt und innerhalb des Gehäuses zu der Steuerungseinheit. Die elektrische Leitung hat somit insbesondere einen in der Halterung geführten (außenliegenden) ersten Abschnitt und einen zweiten (innenliegenden) Abschnitt, der auf der Seite des Gehäuseabschnitts angeordnet ist, der der Halterung gegenüberliegt. Der erste und zweite Abschnitt können separat ausgebildet sein und über einen Anschluss des Gehäuseabschnitts miteinander verbunden oder verbindbar sein.

Die der Erfindung zugrunde liegende Aufgabe wird zudem durch eine extrakorporale Blutbehandlungsmaschine, insbesondere Dialysemaschine, mit einer vorstehend beschriebenen Druckmessanordnung gelöst. Insbesondere hat die Blutbehandlungsmaschine einen Gehäuseabschnitt, insbesondere mit einer zum Gehäuseäußeren gewandten Oberfläche, an welchem bzw. welcher die Halterung befestigt ist

Zusammenfassend wird die der Erfindung zugrunde liegende Aufgabe gelöst durch eine am Gehäuse der Maschine (extrakorporalen Behandlungsmaschine) angebrachte Halterung, z.B. in Form eines Schwanenhalses, welche bevorzugt 15 bis 30 cm lang ist. Ein Druckaufnehmer (Drucksensor) wird am Ende der Halterung angebracht. Der Druck kann somit direkt am POD (Druckabnehmerkapsel) gemessen werden. Die elektrischen Signale werden über Leitungen in der Halterung zu einer Steuerungs- und Kontrolleinheit (Elektronik) übermittelt. Dies hat von Vorteil, dass die Anzahl der Schnittstellen verringert ist, dass der Verbindungsschlauch am POD eingespart werden kann und dadurch Kosten verringert werden können, dass das Totvolumen in sonst vorhandenen Schläuchen verringert wird und dass keine Tiefpasswirkung der Luftstrecke in einem sonst vorhandenen Schlauch vorhanden ist, wodurch der Sensor kürzere Reaktionszeiten auf Druckänderungen auf der Blutseite hat. Der POD kann mittels der Halterung, die bevorzugt in Form eines Schwanenhalses ausgeführt ist, an eine definierte Position gebracht werden. Durch diese Halterung kann die Position des PODs an verschiedene Bruchschlauchleitungen angepasst werden.

Alternativ zur POD-Verbindung (Verbindung zwischen Druckabnehmerkapsel und Drucksensor) mittels Luer kann eine rastende Klick-Verbindung gewendet werden. Die Abdichtung kann dann vorzugsweise radial erfolgen. Im Drucksensor oder an der starren, biegesteifen Fluidleitung kann zusätzlich ein Ventil verbaut sein, um den POD belüften zu können. Dies ist erforderlich, wenn die POD-Membran in eine bestimmte Stellung gebracht werden soll. Dazu kann das Ventil geöffnet werden und die Membran kann durch Druckänderung auf der Blutseite ausgerichtet werden. Nach diesem Vorgang wird das Ventil wieder hermetisch geschlossen. Anstelle der semi-flexiblen Halterung, z.B. in Form eines Schwanenhalses kann auch eine feste Verbindung, die aus der Maschine heraussteht, vorgesehen werden.

### Figurenbeschreibung

Nachfolgend wird die vorliegende Erfindung anhand bevorzugter Ausführungsformen beschrieben. Diese sind jedoch nur veranschaulichender Natur und sollen den Schutzumfang der vorliegenden Erfindung nicht einschränken. Ferner werden bei der Beschreibung der verschiedenen Ausführungsformen für gleiche Bauteile dieselben Bezugszeichen verwendet, um redundante Beschreibungen derselben zu vermeiden.
Fig. 1 zeigt eine erfindungsgemäße Druckmessanordnung einer ersten Ausführungsform der Erfindung mit einem daran angeschossenen Druckabnehmer.
Fig. 2 zeigt eine Detailansicht einer Kopplung des Druckabnehmers mit der Druckmessanordnung der ersten Ausführungsform.
Fig. 3 stellt schematisch eine Druckmessanordnung nach einer zweiten Ausführungsform der Erfindung dar.
Fig. 4 zeigt weitere Aspekte der Druckmessanordnung nach der ersten oder zweiten Ausführungsform.

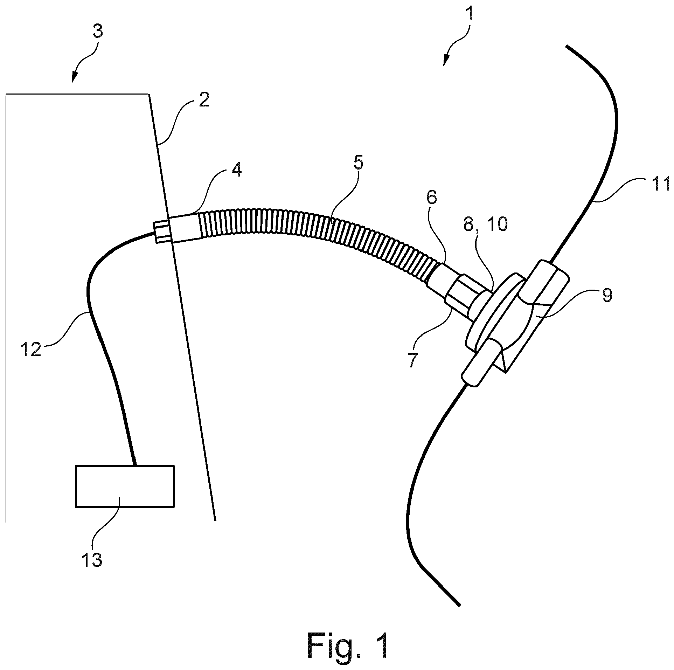

Fig. 1 zeigt eine erfindungsgemäße Druckmessanordnung 1 nach einer ersten Ausführung der Erfindung. An einem den ortsfesten Untergrund bildenden Gehäuse 2 einer extrakorporalen Blutbehandlungsmaschine 3 ist über eine Befestigungsvorrichtung 4 eine Halterung 5 angebracht. Die Halterung 5 ist stabartig oder rohrartig geformt und erstreckt sich von dem Gehäuse 2 nach außen. Genauer ausgedrückt, bildet die Halterung 5 nach dieser Ausführungsform einen Schwanenhals. Ein Schwanenhals ist ein halbsteifes, (manuell / plastisch) biegsames oder abkrümmbares, insbesondere (elastisch) biegestarres Verbindungselement, das manuell in nahezu jede Richtung gebogen werden und in einer entsprechend eingestellten Stellung verharren kann. In Fig. 1 ist schematisch einen Schwanenhals aus einem gewendelten Metallschlauch dargestellt.

An einer dem Gehäuse 2 abgewandten Seite bzw. einem der Befestigungsvorrichtung 4 entgegengesetzten Axialendabschnitt bildet die Halterung 5 eine Anlenkstelle 6 aus. An dieser Anlenkstelle 6 ist ein Drucksensor 7 montiert. Der Drucksensor 7 hat einen Koppelabschnitt 8, durch welchen er an einer Druckabnehmerkapsel 9 angeschlossen ist, welche einen Druckabnehmer bildet. Der Koppelabschnitt 8 bildet ferner eine sehr kurze, starre, biegesteife Fluidleitung 10, welche den Drucksensor 7 mit der Druckabnehmerkapsel 9, genauer mit einer Luftkammer der Druckabnehmerkapsel 9, verbindet. Die Luftkammer ist durch eine Membran von einer Leitungskammer getrennt, welche in einem Leitungssystem 11, insbesondere einer Blutschlauchleitung, der extrakorporalen Blutbehandlungsmaschine 3 integriert oder montiert ist.

Während eines Betriebs der extrakorporalen Blutbehandlungsmaschine 3 liegt in der Leitungskammer der Druckabnehmerkapsel 9 ein Leitungsinnendruck an, welcher über die Membran auf die Luftkammer übertragen wird. Dadurch wird in der Luftkammer ein Fluiddrucksignal gebildet, welches durch den Koppelabschnitt 8 bzw. durch die starre, biegesteife Fluidleitung 10 unmittelbar an dem Drucksensor 7 anliegt und welches durch den Drucksensor 7 in ein elektrisches Signal gewandelt wird. An dem Drucksensor 7 ist zur Übertragung des Druckmesssignals zumindest ein Kabel 12 als eine elektrische Leitung angeschlossen. Das Kabel verläuft durch die rohrartig ausgebildete Halterung 5 oder den Schwanenhals und tritt an einer Montagestelle, an welcher die Befestigungsvorrichtung 4 der Halterung 5 an dem Gehäuse 2 montiert ist, in das Gehäuse 2 ein. Das heißt, das Kabel 12 verläuft derart durch die rohrartige Halterung 5, dass es zwischen dem Drucksensor 7 bis zu dem Gehäuse 2 vollständig von der Umgebung geschützt ist. Das Kabel 12 kann folglich nicht durch Umgebungseinflüsse wie ungeschickte Anwender, aggressive Desinfektionsmittel, etc. beschädigt werden. Zugleich ist die Verstellbarkeit des Drucksensors 7 durch den schwanenhalsartigen Aufbau der Halterung 5 gewährleistet. Innerhalb des Gehäuses 2 der extrakorporalen Blutbehandlungsmaschine 3 ist das Kabel 12 an einer Steuerungseinheit oder einer Elektronik 13 angeschlossen, welche das Signal des Drucksensors 7 empfängt und verarbeitet.

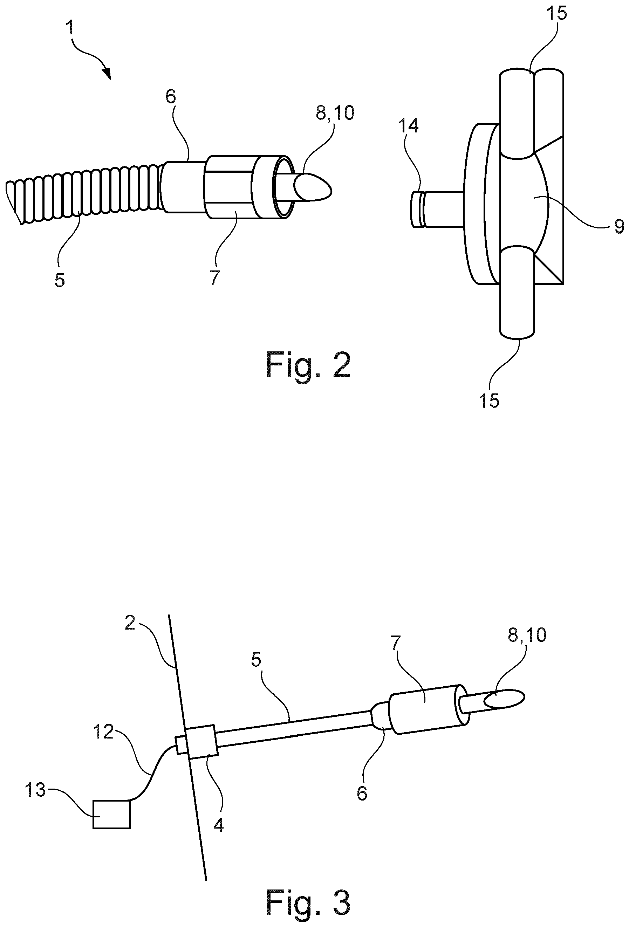

Fig. 2 zeigt eine Detailansicht der den Drucksensor 7 haltenden Anlenkstelle 6 der Halterung 5 während eines Kopplungsvorgangs mit der Druckabnehmerkapsel 9. In dieser Ansicht ist gut erkennbar, dass der Koppelabschnitt 8 der Druckmessanordnung 1, welcher an dem Drucksensor 7 angeordnet ist, nach einem Aspekt der Erfindung als ein männlicher oder weiblicher Luer-Slip/Lock-Konnektor 8 ausgebildet ist. Dementsprechend weist die Druckabnehmerkapsel 9 zur Kopplung an dem Koppelabschnitt 8 an einer Seite der Luftkammer einen Gegenkoppelabschnitt 14 in Form eines weiblichen bzw. männlichen Luer-Slip/Lock-Konnektors auf. Ferner sind zwei mit der Leitungskammer der Druckabnehmerkapsel 9 fluidverbundene Leitungsanschlüsse 15 bereitgestellt, über welche die Druckabnehmerkapsel 9 in die Blutschlauchleitung 11 der extrakorporalen Blutbehandlungsmaschine 3 fluidleitend integriert oder montierbar ist.

Fig. 3 zeigt schematisch eine Druckmessanordnung 1 nach einer zweiten Ausführungsform der Erfindung. Diese zweite Ausführungsform entspricht in ihrem grundlegenden Aufbau der ersten Ausführungsform, weshalb nachfolgend nur auf deren Unterschiede eingegangen wird. Nach der zweiten Ausführungsform weist die Halterung 5 einen starren Stab anstelle eines manuell abkrümmbaren Schwanenhalses auf. In Fig. 3 ist der Stab beispielhaft linear verlaufend dargestellt. Abhängig vom konkreten Aufbau der extrakorporalen Blutbehandlungsmaschine, insbesondere der Anordnung der verschiedenen Leitungen und Gehäusedurchführungen, kann die Halterung 5 bzw. der starre Stab aber auch gekrümmt und/oder geknickt ausgeführt sein, um einen für den konkreten Aufbau optimal angepassten Verlauf zu erreichen.

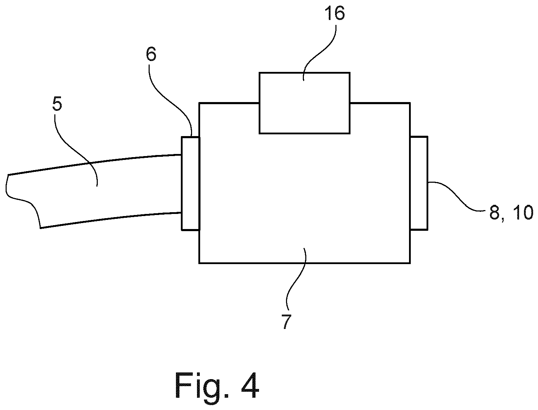

Fig. 4 zeigt schematisch ein Anlenkstelle 6 einer erfindungsgemäßen Druckmessanordnung 1 nach der ersten oder zweiten Ausführungsform, um weitere Aspekte der Erfindung hervorzuheben. Nach einem ersten Aspekt weist der Drucksensor 7 als den Koppelabschnitt 8 anstelle eines Luer-Slip/Lock-Konnektors einen alternativen Konnektor wie z.B. ein klick- oder einrastbares Konnektorelement (hier nur schematisch dargestellt) auf. Nach einem zweiten Aspekt, welcher zusätzlich oder alternativ zu dem ersten Aspekt in beiden der vorstehend beschriebenen Ausführungsformen bereitgestellt werden kann, weist der Drucksensor 7 oder die starre, biegesteife Fluidleitung 10 ein darin integriertes oder montiertes Ventil 16 auf, über welches die Luftkammer der Druckabnehmerkapsel 9 be- oder entlüftet werden kann, um die Membran der Druckabnehmerkapsel 9 in eine bestimmte Stellung zu bringen.

### Referenzzeichenliste

- 1: Druckmessanordnung
- 2: Ortsfester Untergrund, Gehäuse
- 3: extrakorporale Blutbehandlungsmaschine (Dialysemaschine)
- 4: Befestigungsvorrichtung
- 5: Halterung (Schwanenhals / starrer Stab)
- 6: Anlenkstelle
- 7: Drucksensor
- 8: Koppelabschnitt (Luer-Slip/Lock-Konnektor / einrastbares Verbindungselement)
- 9: Druckabnehmer, Druckabnehmerkapsel
- 10: Starre, biegesteife Fluidleitung
- 11: Leitungssystem (Blutschlauchleitung)
- 12: Elektrische Leitung (Kabel)
- 13: Elektronik, Steuerungseinheit
- 14: Gegenkoppelabschnitt
- 15: Leitungsanschlüsse
- 16: Ventil

## Patentansprüche

1. Druckmessanordnung (1) zur Messung des Leitungsinnendrucks eines vorzugsweise extrakorporalen Leitungssystems (11) einer extrakorporalen Blutbehandlungsmaschine (3), insbesondere Dialysemaschine, mit
einem Drucksensor (7), der über eine starre, biegesteife Fluidleitung (10), vorzugsweise Gasleitung, mit einem an das Leitungssystem (11) angeschlossenen oder anschließbaren Druckabnehmer (9) verbindbar ist und ein Fluiddrucksignal aus der starren, biegesteifen Fluidleitung (10) in ein elektrisches Signal wandelt,
einer elektrische Leitung (12), vorzugsweise Kabel, die dazu dient, den Drucksensor (7) mit einer Elektronik (13) zu verbinden, um das elektrische Signal zu verarbeiten, sowie
einer Halterung (5) zum direkten oder indirekten Halten des Drucksensors (7),
**dadurch gekennzeichnet, dass** die Halterung (5) zumindest abschnittsweise einen Innenkanal ausbildet, in welchem die elektrische Leitung (12) in vor der Umgebung geschützter Weise geführt ist.

2. Druckmessanordnung (1) nach Anspruch 1, **dadurch gekennzeichnet, dass** die Halterung (5) als ein im Wesentlicher plastisch abkrümmbarer Stab ausgebildet ist, der an einem Axialendabschnitt eine Befestigungsvorrichtung (4) zur Montage an einem ortsfesten Untergrund (2) und an dem anderen Axialendabschnitt eine Anlenkstelle (6) für den Drucksensor (7) oder den Druckabnehmer (9) aufweist.

3. Druckmessanordnung (1) nach einem der Ansprüche 1 und 2, **dadurch gekennzeichnet, dass** die Halterung (5) den Drucksensor (7) hält und
der Drucksensor (7) die starre, biegesteife Fluidleitung (10) hält oder daran, insbesondere über einen Koppelabschnitt (8), befestigt ist und die Fluidleitung (10) dazu eingerichtet ist, an dem Druckabnehmer (9) befestigt zu werden.

4. Druckmessanordnung (1) nach einem der vorstehenden Ansprüche, **dadurch gekennzeichnet, dass** die Halterung (5) dazu eingerichtet ist, insbesondere außenseitig, an einem Gehäuseabschnitt (2) der extrakorporalen Blutbehandlungsmaschine (3) befestigt zu werden.

5. Druckmessanordnung (1) nach einem der vorstehenden Ansprüche,
**dadurch gekennzeichnet, dass** die Fluidleitung (10) an dem Drucksensor (7) angeordnet ist und durchgängig starr und biegesteif ausgebildet ist.

6. Druckmessanordnung (1) nach einem der vorstehenden Ansprüche,
**dadurch gekennzeichnet, dass** der Innenkanal einen zur Umgebung hin vollständig geschlossenen Aufnahmeraum zur Aufnahme der elektrischen Leitung (12) bildet.

7. Druckmessanordnung (1) nach einem der vorstehenden Ansprüche,
**dadurch gekennzeichnet, dass** die Halterung (5) derart plastisch verformbar oder abkrümmbar ist, vorzugsweise in Form eines Schwanenhalses, ausgebildet ist, sodass eine Position der Anlenkstelle (6) vorzugsweise manuell einstellbar ist.

8. Druckmessanordnung (1) nach Anspruch 7, **dadurch gekennzeichnet, dass** die Halterung (5) als Schwanenhals ausgebildet ist, welcher den Innenkanal bildet.

9. Druckmessanordnung (1) nach einem der Ansprüche 1 und 3 bis 6,
**dadurch gekennzeichnet, dass** die Halterung (5) ein starrer, in einer vorbestimmten, linearen oder gekrümmten Form verlaufender, vorzugsweise hohler Stab ist.

10. Druckmessanordnung (1) nach einem der vorstehenden Ansprüche,
**dadurch gekennzeichnet, dass** der Druckabnehmer nach Art einer Druckabnehmerkapsel (9) ausgebildet ist, die eine erste, mit dem Leitungssystem (11) verbundene oder verbindbare Kammer und eine zweite, vorzugsweise gasgefüllte Kammer aufweist, welche von der ersten Kammer durch eine Membran getrennt ist und an welche der Drucksensor (7) gekoppelt oder koppelbar ist.

11. Druckmessanordnung (1) nach Anspruch 10, **dadurch gekennzeichnet, dass** an dem Drucksensor (7) oder an der starren, biegesteifen Fluidleitung (10) integriert oder montiert ein Ventil (16) bereitgestellt ist, um die Druckabnehmerkapsel (9), insbesondere die zweite Kammer, zu be- oder entlüften.

12. Druckmessanordnung (1) nach einem der vorstehenden Ansprüche,
**dadurch gekennzeichnet, dass** der Drucksensor (7) als einen Koppelabschnitt (8) zum unmittelbaren Koppeln des Drucksensors (7) mit dem Druckabnehmer (9) einen Luer-Anschluss oder ein einrastbares Verbindungselement aufweist.

13. Extrakorporale Blutbehandlungsmaschine (3), insbesondere Dialysemaschine, mit einer Druckmessanordnung (1) nach einem der vorstehenden Ansprüche.

14. Extrakorporale Blutbehandlungsmaschine (3) nach Anspruch 13,
**gekennzeichnet durch** einen Gehäuseabschnitt (2), insbesondere eine zum Gehäuseäußeren gewandte Oberfläche des Gehäuseabschnitts (2), an welchem bzw. welcher die Halterung (5) befestigt ist.

## Claims

1. A pressure-measuring assembly (1) for measuring the internal line pressure of a preferably extracorporeal line system (11) of an extracorporeal blood treatment machine (3), in particular a dialysis machine, comprising
a pressure sensor (7) which is connectable via a rigid, bending resistant fluid line (10), preferably a gas line, to a pressure receiver (9) connected or connectable to the line system (11) and which converts a fluid pressure signal from the rigid, bending resistant fluid line (10) into an electrical signal,
an electric line (12), preferably a cable, which serves to connect the pressure sensor (7) to electronics (13) for processing the electric signal, and
a retainer (5) for directly or indirectly holding the pressure sensor (7),
**characterized in that** the retainer (5) forms, at least in sections, an inner channel in which the electric line (12) is guided such that it is protected from the environment.

2. The pressure-measuring assembly (1) according to claim 1, **characterized in that** the retainer (5) is formed as a substantially plastically bendable rod having, at one axial end portion, a fastening device (4) for being mounted to a stationary base (2) and, at the other axial end portion, an articulation site (6) for the pressure sensor (7) or the pressure receiver (9).

3. The pressure-measuring assembly (1) according to one of claims 1
and 2, **characterized in that** the retainer (5) holds the pressure sensor (7), and the pressure sensor (7) holds or is attached to the rigid, bending resistant fluid line (10), in particular via a coupling portion (8), and the fluid line (10) is adapted to be attached to the pressure receiver (9).

4. The pressure-measuring assembly (1) according to one of the preceding claims, **characterized in that** the retainer (5) is adapted to be attached, in particular on the outside, to a housing portion (2) of the extracorporeal blood treatment machine (3).

5. The pressure-measuring assembly (1) according to one of the preceding claims, **characterized in that** the fluid line (10) is arranged on the pressure sensor (7) and is designed to be rigid and bending resistant throughout.

6. The pressure-measuring assembly (1) according to one of the preceding claims, **characterized in that** the inner channel forms a receiving space for receiving the electrical line (12) that is completely closed with respect to the environment.

7. The pressure-measuring assembly (1) according to one of the preceding claims, **characterized in that** the retainer (5) is plastically deformable or curvable, preferably in the form of a gooseneck, in such a way that a position of the articulation site (6) is preferably manually adjustable.

8. The pressure-measuring assembly (1) according to claim 7, **characterized in that** the retainer (5) is configured as a gooseneck which forms the inner channel.

9. The pressure-measuring assembly (1) according to one of claims 1 and 3 to 6, **characterized in that** the retainer (5) is a rigid, preferably hollow rod extending in a predetermined linear or curved shape.

10. The pressure-measuring assembly (1) according to one of the preceding claims, **characterized in that** the pressure receiver is configured in the manner of a pressure-receiver capsule (9), which comprises a first chamber connected or connectable to the line system (11) and a second, preferably gasfilled chamber, which is separated from the first chamber by a diaphragm and to which the pressure sensor (7) is coupled or coupleable.

11. The pressure-measuring assembly (1) according to claim 10, **characterized in that** a valve (16) is provided integrated or mounted on the pressure sensor (7) or on the rigid, bending resistant fluid line (10) in order to vent the pressure-receiver capsule (9), in particular the second chamber.

12. The pressure-measuring assembly (1) according to one of the preceding claims, **characterized in that** the pressure sensor (7) has, as a coupling portion (8) for directly coupling the pressure sensor (7) to the pressure receiver (9), a Luer connection or a latchable connecting element.

13. The extracorporeal blood treatment machine (3), in particular dialysis machine, with a pressure-measuring assembly (1) according to one of the preceding claims.

14. The extracorporeal blood treatment machine (3) according to claim 13, **characterized by** a housing portion (2), in particular a surface of the housing portion (2) facing the outside of the housing, to which the retainer (5) is attached.

## Revendications

1. Agencement de mesure de pression (1) pour mesurer la pression intérieure de conduite d'un système de conduites (11) de préférence extracorporel d'une machine de traitement extracorporel de sang (3), en particulier d'une machine de dialyse, avec
un capteur de pression (7) qui peut être relié via une conduite de fluide (10) rigide et résistante à la flexion, de préférence une conduite de gaz, à un réducteur de pression (9) qui est ou peut être raccordé au système de conduites (11) et transforme un signal de pression de fluide, en provenance de la conduite de fluide (10) rigide et résistante à la flexion, en un signal électrique,
une conduite électrique (12), de préférence un câble, qui sert à relier le capteur de pression (7) à une électronique (13) pour travailler le signal électrique, ainsi que
un élément de maintien (5) pour maintenir directement ou indirectement le capteur de pression (7),
**caractérisé en ce que** l'élément de maintien (5) constitue au moins par endroits un canal intérieur dans lequel la conduite électrique (12) est guidée de manière à protéger de l'environnement.

2. Agencement de mesure de pression (1) selon la revendication 1, **caractérisé en ce que** l'élément de maintien (5) est conçu en tant que barre essentiellement en plastique pouvant être incurvée, laquelle présente, au niveau d'une section d'extrémité axiale un dispositif de fixation (4) pour le montage sur un support de base (2) stationnaire et au niveau de l'autre section d'extrémité axiale, un emplacement d'articulation (6) pour le capteur de pression (7) ou le réducteur de pression (9) .

3. Agencement de mesure de pression (1) selon l'une des revendications 1 et 2, **caractérisé en ce que** l'élément de maintien (5) maintient le capteur de pression (7) et
le capteur de pression (7) maintient la conduite de fluide (10) rigide et résistante à la flexion ou est fixé contre celle-ci, en particulier via une section de bielle (8), et la conduite de fluide (10) est aménagée pour être fixée sur le réducteur de pression (9).

4. Agencement de mesure de pression (1) selon l'une des revendications précédentes, **caractérisé en ce que** l'élément de maintien (5) est aménagé, en particulier du côté extérieur, pour être fixé sur une section de logement (2) de la machine de traitement extracorporel de sang (3).

5. Agencement de mesure de pression (1) selon l'une des revendications précédentes, **caractérisé en ce que** la conduite de fluide (10) est agencée contre le capteur de pression (7) et conçue constamment rigide et résistante à la flexion.

6. Agencement de mesure de pression (1) selon l'une des revendications précédentes, **caractérisé en ce que** le canal intérieur constitue un espace de réception complètement fermé à l'environnement pour recevoir la conduite électrique (12).

7. Agencement de mesure de pression (1) selon l'une des revendications précédentes, **caractérisé en ce que** l'élément de maintien (5) peut être déformé ou incurvé plastiquement, de préférence sous la forme d'un col de cygne, conçu de sorte qu'une position de l'emplacement d'articulation (6) puisse être réglée de préférence manuellement.

8. Agencement de mesure de pression (1) selon la revendication 7, **caractérisé en ce que** l'élément de maintien (5) est conçu en tant que col de cygne, lequel constitue le canal intérieur.

9. Agencement de mesure de pression (1) selon l'une des revendications 1 et 3 à 6, **caractérisé en ce que** l'élément de maintien (5) est une barre rigide qui se déroule selon une forme prédéterminée, linéaire ou incurvée, de préférence creuse.

10. Agencement de mesure de pression (1) selon l'une des revendications précédentes, **caractérisé en ce que** le réducteur de pression est conçu à la manière d'une capsule de réducteur de pression (9) qui présente une première chambre qui est ou peut être reliée au système de conduites (11), et une seconde chambre de préférence emplie de gaz, laquelle est séparée de la première chambre par une membrane et au niveau de laquelle le capteur de pression (7) est ou peut être couplé.

11. Agencement de mesure de pression (1) selon la revendication 10, **caractérisé en ce qu'**un clapet (16) est prévu, intégré ou monté au niveau du capteur de pression (7) ou au niveau de la conduite de fluide (10) rigide et résistante à la flexion, pour ventiler ou purger la capsule de réducteur de pression (9), en particulier la seconde chambre.

12. Agencement de mesure de pression (1) selon l'une des revendications précédentes, **caractérisé en ce que** le capteur de pression (7) présente, en tant que section de bielle (8) pour un accouplement immédiat du capteur de pression (7) avec le réducteur de pression (9), un raccord Luer ou un élément de liaison qui peut être encliqueté.

13. Machine de traitement extracorporel de sang (3), en particulier machine de dialyse, avec un agencement de mesure de pression (1) selon l'une des revendications précédentes.

14. Machine de traitement extracorporel de sang (3) selon la revendication 13, **caractérisé par** une section de logement (2), en particulier une surface tournée vers l'extérieur de logement de la section de logement (2) contre laquelle l'élément de maintien (5) est fixé.
